# EUROPEAN PATENT APPLICATION

(11) **EP 1 481 669 A1**
(43) Date of publication of application: **01.12.2004**
(21) Application number: 03012122.2
(22) Date of filing: 30.05.2003
(51) Int. Cl.: A61K 31/05, A61K 31/192, A61K 31/352, A61K 35/78, A61K 9/127

(54) **Use of polyhydroxy phenols and polyphenols for modulating p-selectin activity**

(71) Applicant: YAMANOUCHI PHARMACEUTICAL CO. LTD., Tokyo 103-8411 (JP)
(72) Inventor: Biessen, Erik Anna Leonardus LACDR Leiden Univ., 2300 RA Leiden (NL); Appeldoorn, Chantal Catharina Maria LACDR, 2300 RA Leiden (NL); Bonnefoy, Arnaud, 300 Leuven (BE); van Berkel, Theodorus Josephus Cornelis LACDR, 2300 RA Leiden (NL); Kuiper, Johan LACDR Leiden Univ., 2300 RA Leiden (NL); Hoylaerts, Marc Florimond, 300 Leuven (BE)
(74) Representative: HOFFMANN - EITLE

(57) **Abstract**

The use of polyhydroxy phenols and polyphenols for the manufacture of a medicament for the prevention, treatment or diagnosis of a disease or a condition, wherein P-selectin is involved, is provided.

Further, pharmaceutical and nutraceutical compositions which are useful in the diagnosis, prevention, or treatment of diseases and conditions associated with P-selectin activity are provided.

Also the use of polyhydroxy phenols and polyphenols as targeting tools to P-selectin expressing cells or tissues in a composition, further comprising an active compound in a vehicle, is provided.

## Description

The present invention relates to the use of polyhydroxy phenols and polyphenols for the preparation of a medicament for the prevention, treatment or diagnosis of diseases or disorders wherein P-selectin is involved. It also relates to compositions containing a polyhydroxy phenol or a polyphenol for use in the prevention, treatment or diagnosis of the said diseases and disorders. In addition thereto the present invention relates to the use of polyhydroxy phenols and polyphenols as a targeting agent to P-selectin-expressing cells.

### BACKGROUND OF THE INVENTION

In recent years, coronary artery disease (CAD) has become a major cause of death in western societies. Hyperlipidemia and atherosclerosis are regarded as major risk mechanisms for coronary diseases, whereas there is some evidence that hypertension, diabetes and excess body weight may have less negative impact on coronary disorders than previously assumed.

Atherosclerosis is thought to be initiated at critical sites of the arterial vasculature by a process of monocyte adhesion to the vessel wall, sustained by the occurrence of active functional changes on the endothelial surface (M.A. Gimbrone et al., Thromb. Haemost. 82(2): 722-6 (1999)).

Blood platelets play a major role in coronary artery disease (B.A. Osterud, Thromb. Res., 85: 1-22, 1997). Platelets are found at the sites of early atherosclerotic lesions. When activated, platelets secrete potent mitogenic factors such as platelet-derived growth factor, transforming growth factor β, and epidermal growth factor, which lead to smooth muscle proliferation and progression of atherosclerotic lesions. Enhanced platelet reactivity and spontaneous platelet aggregation were associated with a higher risk of recurrent coronary artery disease. Physiologic anti-platelet metabolites, such as nitric oxide, activate platelet guanylate cyclase and elevate cyclic guanosine-3',5'-monophosphate, thereby reducing fibrinogen binding to the glycoprotein IIb-IIIa (GPIIb-IIIa) receptor through inhibition of agonist-mediated calcium flux.

Several interventions demonstrated a decreased risk of cardiovascular disease, such as with therapeutic doses of aspirin (R. Collins et al., N. Eng. J. Med., 336: 847-60, 1997) and antioxidant supplements (C.H. Hennekens, Am. Heart J., 128: 1333-6, 1994). Therapy with anti-platelet agents such as aspirin and clopidogrel significantly decreases the incidence of primary and secondary coronary events (C.H. Hennekens, Annu. Rev. Public Health 18: 37-49, 1997). Antibodies and peptides that block the fibrinogen binding to activated platelet glycoprotein IIb-IIIa have improved the results of coronary revascularisation procedures. Activation-dependent platelet antigens also indicate changes in platelet function after physical exercise, physiologic challenges (D. Rajasekhar, Thromb. Haemost. 77: 1002-7, 1997), and dietary intervention (M.A. Allman-Farinelli et al., Thromb. Res., 90: 163-9, 1998).

In recent years, certain dietary components, notably unsaturated fatty acids and polyphenols, have been identified as key mediators in numerous cellular processes. As observed by De Caterina et al. (Atherioscler. Thromb., 14: 1829-36, 1994; Prostaglandins Leukot. Essent. Fatty Acids, 52: 191-5, 1995), ω-3 fatty acids, which have effects on total and LDL cholesterol and yet also appear to be linked to protection from atherosclerosis, may act by inhibiting early atherogenic events related to monocyte adhesion to endothelial cells. This process occurs through inhibition of endothelial activation, i.e. the concerted expression of cytokine-inducible endothelial leukocyte adhesion molecules and leukocyte chemo-attractants affecting monocyte adhesion. Inhibition of a common signal-transduction pathway involving the transcription factor nuclear factor-κB (NF-κB) was therefore suggested.

The involvement of nuclear factor-κB has been established by M.A. Carluccio et al. (Atherioscler. Thromb. Vasc. Biol., 23(4): 622-629, 2003). They demonstrated that oleuropein and hydroxytyrosol (major components of olive leaf extract) inhibit at nutritionally relevant concentrations transcriptional endothelial adhesion molecule expression, thus possessing atheroprotective features.

Several epidemiological and laboratory studies indicate that moderate alcohol consumption can lower the risk of atherosclerosis and hyperlipidemia and thus the chance of coronary artery disease (E.B. Rimm et al., BMJ, 312: 731-36, 1996; D.M. Goldberg et al., Clin. Chim. Acta, 237: 155-187, 1995). In France, where the red wine consumption is relatively high as compared to Northern European Countries and the USA, but the pattern of saturated fat intake similar, the mortality rate of CAD is approximately 50 % lower. This so called "French Paradox" (S. Renaud et al., Lancet, 339: 1523-1526, 1992) has been attributed to the major constitute of "wine tannins" the polyphenolic alcohols, such as gallic acid and flavonoids of the catechin family including (+)-catechin, (-)-epicatechin, and procyanidin B2, which are abundantly present in red wine (S. Rosenkranz et al., Faseb J., 16: 1958-1960, 2002; P.M. Kris-Etherton et al., Am. J. Med., 113, Suppl. 9B: 71-88, 2002).

Phenolic substances contained in red wine have been found to inhibit oxidation of human LDL and thus postulated to possess atheroprotective properties. However, it is difficult to attribute a reduction of atherosclerotic processes and consequently protection from coronary artery disease only to the inhibition of LDL oxidation because many vascular effects of antioxidants are not related to the resistance of LDL to oxidation (M.N. Diaz et al., N. Eng. J. Med., 337: 408-416, 1997).

Several studies carried out in humans and animals have shown that wine phenolic compounds could exert their effects by reducing prostanoid synthesis from arachidonate. In addition, it has been suggested that wine phenolic fractions could reduce platelet activity mediated by nitric oxide. Moreover, wine phenolic components increase vitamin E levels, while decreasing the oxidation of platelets submitted to oxidative stress.

M.E. Ferrero et al. (Am. J. Clin. Nutr., 68: 1208-14, 1998) showed the role of resveratrol (a polyphenol present in red wine) in the regulation of the endothelial cell adhesion molecule-1 expression, and thus demonstrated that its antiatherogenic activity is not in the front rank related to the protection of LDL from oxidation as postulated by M.N. Diaz et al. (ibid).

S. Rotondo et al. (Br. J. Pharmacol., 123 (8): 1691-99, 1998) investigated the effect of trans- resveratrol on functional and biochemical responses of polymorphonuclear leukocytes (PMN), which are suggested to be involved in the pathogenesis of acute coronary heart diseases. The results of their studies indicate that trans-resveratrol interferes with the release of inflammatory mediators by activated PMN and down-regulates adhesion-dependent thrombogenic PMN functions.

M.A. Carluccio et al. (Atherioscler. Thromb. Vasc. Biol., 23(4): 622-29, 2003) confirmed recently the conclusions by Ferrero et al. and S. Rotondo et al. establishing the involvement of nuclear factor-κB as key transcription factor for the endothelial cell adhesion molecule-1 expression.

D. Rein et al. (Am. J. Clin. Nutrition, 72(1): 30-35, 2000; J. of Nutrition, 130 (8S): 2120S-2126S, 2000) executed a series of in vitro and in vivo studies on the effects of cocoa procyanidins (trimers and pentamers), epicatechin and de-alcoholised wine (DRW) on platelet activation. Fluorescent-labeled monoclonal antibodies recognizing the fibrinogen binding conformation of GPIIb-IIIa (PAC-1 binding) and the activation-dependent platelet epitope CD62P (P-selectin) were selected as markers for the platelet activation. Both tested components added to whole blood in vitro increased PAC-1 binding and P-selectin expression on unstimulated platelets, but suppressed the platelet activation in response to epinephrine. In contrast, cocoa procyanidins inhibited stimulated platelet activation in whole blood, whereas the effect of de-alcoholised wine was not that much pronounced. Generally, this suppressive effect observed on platelet reactivity may explain the cardioprotective effects of polyphenols present in wine or other nutritional preparations (e.g. cocoa beverages and chocolate).

Red wine polyphenols and their impact on platelet aggregation have been moreover studied by P. Russo et al. (Nutr. Metab. Cardiovasc. Dis., 11 (1): 25-9, 2001). They have isolated four classes of wine phenolic compounds: phenolic acids (fraction 1), procyanidins, catechins and monomeric anthocyanidins (fraction 2), flavonols and resveratrol (fraction 3) and polymeric anthocyanidins (fraction 4). The effect of each fraction on ADP-induced platelet aggregation in rats and c-AMP content was compared with that of de-alcoholised red wine (DRW) and the pure phenolic compounds alone (quercetin, catechin, resveratrol, caffeic acid). Both DRW and the phenolic fraction 2 inhibited significantly ADP-induced platelet aggregation, whereas the effects of fractions 3 and 4 and the pure phenolic compounds were not significant. A significant increase in platelet c-AMP content was observed first after the addition of DRW and fraction 2.

As another approach to exhibit the impact of polyphenols in red wine on the platelet activation and consequently their atheroprotective properties, explored A.D. Blann et al. (Blood Coagul. Fibrinolysis, 13 (7): 647-651 (2002)) markers of platelet activity (beta-thromboglobulin and soluble P-selectin) and endothelial cell function (von-Willebrand-factor and soluble thrombomodulin) before and upon ingestion of red wine in vivo. The only significantly increase was noticed for beta-thromboglobulin. The study results led to the conclusion that red wine activates the platelets without having any substantial effect on the endothelium.

Rosenkranz et al. (Faseb J., 16: 1958-1960, (2002)) observed that non-alcoholic constituents of red wine that accumulate during mash fermentation act as potent inhibitors of platelet-derived growth factor β (βPDGFR) signalling and PDGF-dependent cellular responses in vascular smooth muscle cells (VSMC). Signals initiated by the βPDGFR play an important role in vascular development and the pathogenesis of atherosclerosis. PDGF-dependent migration and proliferation of VSMC are critical steps during atherogenesis. In the same work they demonstrated that mainly the flavonoids of the catechin family inhibit the PDGF-dependent tyrosine phosphorylation of the βPDGFR, whereas gallic acid only does not mediate a significant effect.

Furthermore, it is known from US 6,133,311 that gallic acid inhibits the activities of 3-hydroxy-3-methyl-glutaryl-Coenzyme A (HMG-CoA reductase), which mediates the synthesis of mevalonic acid, an intermediate in the biosynthesis of sterols, e.g. cholesterol, or isoprenoids (William W. Parmley and Kanu Chatterjee (Eds.), Cardiovascular Pharmacology, Wolfe Publishing, 1994). Thus, it reduces the rate of cholesterol biosynthesis and prevents therefore arteriosclerosis and hypercholesterolemia which are known to be strongly related to CAD.

In US 6,133,311, an additional mechanism of action is proposed for gallic acid when used to control increased plasma cholesterol level. They are referring to an interaction of gallic acid with the acyl CoA-cholesterol-o-acyltransferase (ACAT). ACAT promotes the esterification of cholesterol in blood. Foam cells are formed by the action of ACAT and contain a large amount of cholesterol ester carried by low density lipoprotein (LDL) in the blood (D.T. Witiak and D.R. Feller (Eds.), Anti-Lipidemic Drugs: Medicinal, Chemical and Biochemical Aspects, Elsevier, pp 159-195 (1991)). The formation of foam cells in the arterial wall increases with the ACAT activity. Accordingly, as gallic acid inhibits the action of ACAT may also lead to prevention of atheriosclerosis and hyperlipidemia.

Notwithstanding the increased understanding of some of the major diseases including coronary artery disease and atherosclerosis, and the availability of some methods to control these diseases and conditions to some degree, there is still a need for further preventive, therapeutic and diagnostic measures. In the light of the recently developed knowledge on the role of P-selectin in many inflammatory and neoplastic diseases, there is also a need for improved methods to control or antagonise the effects of P-selectin activation in humans. Furthermore, there is a need for such methods which reduce the risk of developing diseases associated with P-selectin activity which are cost-effective, acceptable to large fractions of the population, safe and tolerable. Finally there is a need for improved methods which can be used for early diagnosis of conditions leading to P-selectin associated diseases.

### SUMMARY OF THE INVENTION

The object of the invention is to provide the use of polyhydroxy phenols and polyphenols for the manufacture of a medicament for the prevention, treatment or diagnosis of a disease or a condition, wherein P-selectin is involved.

A further object of the invention is to provide pharmaceutical and nutraceutical compositions which are useful in the diagnosis, prevention, or treatment of diseases and conditions associated with P-selectin activity.

Another object is to provide the use of polyhydroxy phenols and polyphenols as targeting tools to P-selectin expressing cells or tissues in a composition, further comprising an active compound in a vehicle.

### DETAILED DESCRIPTION OF THE INVENTION

In a first aspect, the invention relates to the use of a polyhydroxy phenol or a polyphenol for the preparation of a medicament for inhibiting the activity of P-selectin in a subject which has developed a disease or condition in which P-selectin is involved or is at risk of developing such disease or condition.

As used herein, inhibition refers to any type of interaction which directly or indirectly leads to the modulation of the biological activity of P-selectin. Common types of inhibition include competitive, uncompetitive, and non-competitive inhibition. Furthermore, inhibition may be described as reversible or irreversible. In practice, many inhibitory processes are of a competitive and reversible nature. Inhibition, in the broad definition used herein, also includes a type of interaction in which an initial phase of activation is observed, followed by a depression of bioactivity. Typically, an inhibitory effect can be found when an inhibitor binds with high affinity to the target molecule, whether the binding takes place in the same molecular region as the natural substrate (i. e. as a mimic) or not. In the case of P-selectin, different binding sites exist for various substrates. For instance, P-selectin glycoprotein ligand-1 (PSGL-1) binding involves a different pocket or site than that of sialyl Lewis X (sLeX). Without wishing to exclude other interactions between polyhydroxy phenols and polyphenols within the scope of the invention, it has been found by the inventors that gallic acid blocks the dynamic interaction between P-selectin and PSGL-1.

The subject to whom the polyhydroxy phenols or polyphenols are to be administered preferably is a human individual. However the compounds in accordance with the invention may, in principle, be applied to other subjects, such as mammals. The effectiveness thereof will depend on the affinity of the polyhydroxy phenols or polyphenols to the P-selectin molecule found in the specific species, as the sequence of this protein differs between species. It has been shown by the inventors, however, that e.g. gallic acid and (-)-epigallocatechin gallate have a high affinity to human P-selectin, and they can be used to effectively modulate its activity, particularly as antagonists or partial antagonists.

Polyhydroxy phenols and polyphenols useful for preparing a medicament for the prevention, treatment or diagnosis of a P-selectin-associated disease or condition include gallic acid, gallic acid derivatives and compounds that are chemically related to gallic acid or include one or more gallic acid moieties. Also included are (precursor) compounds which, after administration, undergo chemical or enzymatic degradation to produce in situ gallic acid, the gallic acid derivative or the compound that is chemically related to gallic acid includes one or more gallic acid moieties. Gallic acid derivatives according to the invention include chemical structures derived from gallic acid, such as conjugates, dimers, multimers, salts, esters, ethers, amides etc. Furthermore, the derivatives include those compounds which differ from gallic acid chemically to some degree, such as by the number and/or position of phenolic hydroxyl groups or by the presence of one or more additional substituents, but which have affinity to P-selectin. Polyphenols are defined as compounds, that include more than one 6 carbon atoms-bearing aromatic ring, having one or more hydroxyl groups attached to it.

Gallic acid, or 3,4,5-trihydroxybenzoic acid, is a natural polyhydroxy phenol found in fruits, vegetables, and herbs, such as in gall nuts, walnuts, mango seeds, red grapes, green tea, and olive oil. In many plant products, gallic acid is contained in the form of precursors such as tannic acid, also named tannin or gallotannin, which describes a class of compounds with a complex and non-uniform chemical structure. Tannins may be divided into 2 groups: (a) derivatives of flavanols, so-called condensed tannins and (b) hydrolysable tannins (the more important group) which are esters of a sugar, usually glucose, with one or more trihydroxybenzenecarboxylic acids. Gallic acid is a major hydrolysis product of tannin.

Gallic acid possesses very low toxicity, which is a significant advantage of the method of the invention compared to other methods using novel inhibitors of P-selectin which tend to be toxic in low doses, or whose physiological tolerability has yet to be established.

Examples of other polyhydroxy phenols are: n-docedyl gallate, caffeic acid and 3,4,5-trihydroxy cinnamic acid. Examples of polyphenols according to the invention are (-)-epigallocatechin gallate, (epi)catechin, m-galloyl gallic acid and ellagic acid.

The use of the polyhydroxy phenols and the polyphenols in accordance with the invention requires that such compounds, having affinity to P-selectin, are administered in an effective amount. With regard to methods of identifying such polyhydroxy phenols and the polyphenols which have affinity to P-selectin, useful examples are given in WO 03/020753, the disclosure of which is incorporated herein by reference. The exact amount or dose which needs to be administered in order to reduce the activity of P-selectin depends on various parameters, but can be determined by methods which are known to professionals skilled in this field. Reference is also made to WO 03/020753 and the literature quoted therein, which contains some information on methods of analysing the activity of P-selectin in an organism. Furthermore, the dose to be administered will, in any individual case, depend on factors such as the weight and age of the individual to whom the active material is to be administered, the severity of the conditions, symptoms, or risks which are to be reduced, the specific anti-P-selectin activity of the active compound or mixture of compounds which is used etc.

Affinity to P-selectin is obviously a quantitative parameter. The polyhydroxy phenols and polyphenols in accordance with the invention have an affinity in the micromolar range, such as expressed by an IC₅₀ value of less than 1000 µM, preferably less than 300 µM. More preferred are derivatives with an affinity in the middle or low micromolar range, such as an IC₅₀ of less than 200 to 100 µM. Another preferred range of affinity is that in the lower micromolar or even submicromolar region, such as expressed by an IC₅₀ of less than 100 to 50 µM, or of less than 10 or even 1 µM.

In the case of gallic acid itself, for instance, it is desirable to achieve levels of the active compound at the site of action which are preferably at least in the micromolar range, i. e. at least 1 µM. Higher concentrations are preferred, such as at least 10 µM. From the evidence presently available it may be suggested that gallic acid levels of 10 µM at the site of action may be quite effective in blocking P-selectin substantially, and doses which lead to such levels at the site of action are therefore preferred according to the invention. On the other hand, long-time studies may in the future also provide additional evidence that certain beneficial effects may be achieved with somewhat lower levels at the site of action, or that particularly beneficial effects may be achieved better with a higher degree of P-selectin achievable with levels of 10 µM or more at the site of action.

If other polyhydroxy phenols or polyphenols having affinity to P-selectin are used instead of gallic acid, it will be easy to the skilled person to determine or calculate dose equivalents for these compounds having the same physiological effect as certain gallic acid concentrations.

In most cases, the desired concentration of the active compound(s) at the site of action, as well as the degree of P-selectin inhibition which is aimed at, will be achieved with doses of more than 1 µg per day. In the case of gallic acid, the preferred dose range is from 1 µg to 10 g per day. In another embodiment, the daily dose is at least 10 mg, and in another embodiment it is at least 100 to 500 mg. Of course, when derivatives with much higher anti P-selectin activity than gallic acid or (-)-epigallocatechin gallate are used, the dose per day which needs to be administered to achieve beneficial effects may be considerably less, such as less than 100 mg, less than 10 mg, or even less than 1 mg.

An important factor associated with the desired degree of P-selectin inhibition is whether the aim of using the compounds in accordance with the invention is to prevent or to treat a condition, symptom, or disease associated with P-selectin activity. Over the past few years, an increasing body of evidence has been generated that P-selectin is indeed associated with a number of pathological processes related to inflammation and cancer, and in particular to inflammatory processes leading to major diseases of the cardiovascular system. Among the conditions which are presently associated with P-selectin are coronary artery disease, thrombosis, cancer, chronic inflammatory disorders, rheumatoid arthritis, inflammatory bowel disease, multiple sclerosis, atherosclerosis, restenosis, ischemia, reperfusion injury including renal failure, tumour metastasis, bacterial sepsis, disseminated intravascular coagulation, adult respiratory stress syndrome, stroke, angiogenesis, transplant rejection, deep vein thrombosis, myocardial infarction and circulatory shock. Some of the mechanisms by which P-selectin may influence these conditions are described in more detail in: D.J. Lefer (Annu. Rev. Pharmacol. Toxicol. 40: 283-294 (2000)); R.W. McMurray (Semin. Arthritis Rheum. 25(4):215-233 (1996))

In a preferred embodiment the use involves the administration of a polyhydroxy phenol or a polyphenol to an individual which has already developed such a P-selectin associated disease, condition, or symptom.

In another preferred embodiment, the use in accordance with the invention involves the administration of a polyhydroxy phenol or a polyphenol to an individual which may or may not already have developed a disease, condition, or symptom associated with P-selectin activity, but which is at risk of developing such. In the broadest sense, any subject, which is not excluded from the risk categories and factors commonly associated with cardiovascular diseases, inflammatory diseases, or neoplastic diseases which together represent the most important causes of death in the western societies, may be at risk and therefore benefit from the use in accordance with the invention. In this sense, such a subject may be a human being, typically a member of one of the western societies, and preferably one who does not already - such as by virtue of any dietary habits - consume significant amounts of natural P-selectin inhibitors.

In another preferred embodiment, the individual is at an increased risk of developing a disease, condition, or symptom associated with P-selectin activity. An increased risk is typically defined as a risk substantially above the average risk of a reference group. Increased risks may be characteristics of individuals or of groups. For instance, individuals with certain personal dietary habits, such as high animal fat consumption, or heavy smokers, may have a substantially increased risk of developing a cardiovascular disease such as coronary artery disease than the average peers. On the other hand, dietary factors influencing the cardiovascular risks may often also by characterised as national traits, such as the consumption of saturated fats and oils in some countries in which unsaturated vegetable oils are traditionally rather uncommon.

At a particularly high risk in the context of the present invention are those individuals who have already developed some symptoms - such as high blood pressure, hyperlipidemia or diabetes - which are frequently considered prodromal for the P-selectin associated diseases listed above. It is preferred that P-selectin inhibition by administering polyhydroxy phenols or polyphenols is performed on such individuals.

The administration of polyhydroxy phenols and polyphenols refers to any way of providing an individual with such compounds, whether by ingestion, also referred to as the (per)oral route of administration, or by any other route, such as mucosal, buccal, sublingual, lingual, nasal, rectal, vaginal, pulmonary, subcutaneous, dermal, transdermal, intramuscular, intraperitoneal, intravenous or intraarterial administration. The most common ways of administration are typically those which are associated with a high degree of consumer or patient preference and compliance. Hence, the most preferred way of administration according to the invention is by ingestion. A large number of product types, formulation techniques and dosage forms are available to suit almost any purpose and active ingredient which can be made bioavailable via the oral route. In another preferred embodiment, the polyhydroxy phenols or polyphenols are administered parenterally, preferably by injection, in particular by intravenous injection. Some exemplifying details on oral and parenteral formulation methods will be given further below.

In another embodiment, the polyhydroxy phenols and polyphenols having affinity to human P-selectin are not (primarily) used as P-selectin antagonists, but as a targeting agent or a homing device, in combination with a drug delivery system. More in particular, this embodiment is a method of delivering an active compound to a P-selectin-associated target within an individual, comprising the administration of a targeted drug delivery system which comprises a vehicle, an effective amount of the active compound, and a polyhydroxy phenol or a polyphenol as a targeting agent. It is also possible to use this system as an imaging tool, whereby the active compound is substituted by a contrast agent or a radionuclide.

The polyhydroxy phenols and polyphenols can be directly coupled to the active compounds that are to be delivered to such targets. Alternatively, they can be incorporated into or anchored onto the surface of larger vehicles, such as liposomes or other lipid vesicles, emulsion droplets, polymers, nano- or microparticles (including nanospheres, nanocapsules, microspheres, microcapsules etc.), hydrogels, complexes, or virosomes to obtain targeted vehicles for drugs or genetic material which is delivered to P-selectin expressing cells or tissues. Also multimeric presentation of the polyhydroxy phenols and polyphenols is to be accompanied by a higher affinity for P-selectin.

In this embodiment, the active compound may or may not be a P-selectin inhibitor. More typically, it will be a compound acting on the targeted cells or tissue via a different mechanism, and the method will typically be applied when P-selectin inhibition alone is not sufficient to produce the desired effect. Chemically, the active compound may represent any class of compounds, such as natural, semisynthetic or synthetic small organic molecules, an inorganic substance, a peptide, a protein, a polysaccharide, or a nucleic acid such as an oligonucleotide, a DNA or RNA. Preferably, the active compound is either a nucleic acid, or it is a compound with any type of inhibitory action on the target cell or tissue.

Target cells expressing P-selectin are platelets and activated endothelial cells.

The invention also relates to pharmaceutical or nutraceutical compositions comprising a polyhydroxy phenol or a polyphenol having affinity to P-selectin.

As used herein, the term pharmaceutical composition refers to therapeutic and diagnostic compositions, as well as to medicaments and diagnostics containing such compositions. Therapeutic compositions and medicaments are used for the prevention or treatment of diseases and other conditions of individuals of which conditions improvement is desirable. Diagnostics and diagnostic compositions are used for the diagnosis of such diseases *in vivo* and *in vitro.* Preferred are therapeutic compositions or medicaments to prevent or improve diseases and conditions involving P-selectin. The compositions can also be used for treating diseases in which the inhibition of P-selectin-mediated intracellular signaling is desirable.

Nutraceutical compositions include all compositions typically understood as functional foods or food additives. Nutraceuticals also comprise products isolated or purified from foods and generally formulated in a fashion similar to pharmaceutical dosage forms not usually associated with food, and which have demonstrated to have a physiological benefit, or provide protection against chronic disease.

Nutraceutical compositions are typically formulated and processed for oral use, while the pharmaceutical compositions containing gallic acid or a gallic acid derivative having affinity to P-selectin may be adapted for various routes of administration, such as oral, parenteral, transmucosal, nasal, or pulmonary. Preferred are compositions for parenteral and oral use, and especially preferred are formulations adapted for oral administration. In this document, the term formulations is used for such nutraceutical and pharmaceutical compositions, and the terms peroral and oral are used interchangeably.

The pharmaceutical compositions preferably contain one or more active compounds as defined above and at least one carrier or excipient. As used herein, a carrier or excipient is any pharmaceutically acceptable substance or mixture of substances having no substantial pharmacological activity, which can be used as a vehicle or as an auxiliary substance to formulate a compound into dosage form which is stable and easy to administer. Examples of pharmaceutically acceptable excipients are found in the monographs of all major pharmacopoeias.

Appropriate dosage forms for peroral administration include solid dosage forms such as tablets, hard capsules, soft capsules, powders, granules, orally disintegrating dosage forms effervescent tablets, chewable tablets, oral films, lyophilised dosage forms. The solid form can provide for immediate, sustained or controlled release of the active compound. In one of the embodiments, the oral dosage form is an enteric-coated solid dosage form to provide protection of the compound from the acidic environment of the stomach. Solid dosage forms may be prepared following conventional manufacturing approaches such as wet granulation, direct compression or simple mixing of the active compound and the excipients. Additionally, liquid dosage forms such as syrups, drops, and suspensions, wherein the active compound is dissolved or suspended respectively, are considered as suitable for this purpose. They may further contain drug targeting agents, bioavailability enhancement agents, or active ingredients other than compounds of the invention.

Excipients that are commonly used for the preparation of solid dosage forms for oral administration are binding agents such as gelatin, natural gums, such as acacia, tragacanth; starches, sodium alginate, sugars, polyvinylpyrrolidone; cellulose derivatives such as hydroxypropylmethyl cellulose, polyvinyloxoazolidones; (pharmaceutical) fillers such as lactose, microcrystalline cellulose, dicalcium phosphate, tricalcium phosphate, calcium sulfate, dextrose, mannitol, sucrose; tableting lubricants if needed, such as calcium and magnesium stearate, stearic acid, talc, sterotex (alkaline stearate); disintegrants such as starch, starch derivatives, and crosslinked polymers.

Essential excipients for the preparation of liquid dosage forms are solvents, cosolvents and other liquid carriers, such as water, ethanol, propylene glycol, polyethylene glycol; substances to increase the viscosity of the vehicle such as sugars, especially glucose, water swellable polymers such as cellulose derivatives (e.g. carboxymethylcellulose sodium) or polyvinylpyrrolidone; stabilizers preventing coagulation and caking of the suspension, and preservatives such as parabens; other excipients for taste masking and taste improvement like sweeteners and flavors.

Surfactants may be used to enhance wetting and dissolution of the active compounds. Exemplary of useful surfactants are sodium lauryl sulfate, sorbitan monolaurate, sorbitan monostearate, polysorbate 80, polysorbate 60, poloxamer 407, poloxamer 188 (polyoxethylene, polyoxypropylene block polymers), polyoxyl 20 cetostearyl ether, dioctyl sodium sulfosuccinate, dioctyl calcium sulfosuccinate, nonoxynol, benzalkonium chloride, sorbitan monooleate.

Stabilizers may be incorporated to prevent the oxidation of gallic acid or gallic acid derivatives during storage, thus extending the shelf life of the composition.

In one embodiment, the compositions are formulated and processed for parenteral administration, preferably for intravascular injection, such as intravenous or intra-arterial, but also for intramuscular, subcutaneous, intralesional, intraperitoneal or other routes of parenteral administration. The formulation and manufacture of such compositions follow the same principles as applied to the formulation of other drug substances for these particular administration routes. As an example, sterility is one of the essential requirements for the preparation of parenteral dosage forms. Other requirements, such as pH value and osmolality, are described in all major pharmacopoeias, such as in USP 24, in the monograph "General Requirements for Tests and Assays. 1. Injections". In order to improve the stability of a parenteral formulation, it may be necessary to provide a dried dosage form which must be reconstituted before it can be administered. An example of such a dosage form is a freeze-dried or lyophilised formulation.

In an attempt to avoid frequent injections and to improve patient's compliance and the convenience of the therapy, it may be desirable to administer the compound of the invention as a parenteral controlled release dosage form. A variety of methods for preparing such depot formulations are extensively described in the literature. Prolonged release may be provided by solid implants, nanoparticles, nanocapsules, microparticles, microcapsules, emulsions, suspensions, oily solutions, liposomes, or similar structures.

Due to tolerability issues, the use of excipients in parenteral formulations is somewhat limited. Nevertheless, excipients that are particularly useful for the preparation of parenteral formulations are solvents, cosolvents and liquid or semisolid carriers, such as sterile water, ethanol, glycerol, propylene glycol, polyethylene glycol, butanediol, fatty oils, short- and medium chain triglycerides, lecithin, polyoxyethylene castor oil derivatives; substances to adjust the osmolality and pH, such as sugars, especially glucose, sugar alcohols, especially mannitol, sodium chloride, sodium carbonate, citric acid, acetate, phosphate, phosphoric acid, hydrochloric acid, sodium hydroxide etc.; stabilizers, antioxidants, and preservatives, such as ascorbic acid, sodium sulfite or -hydrogen sulfite, EDTA, benzyl alcohol etc.; other excipients and lyophilisation aids, such as albumin, dextran and the like.

If an active compound, such as a polyphenol with affinity to P-selectin as disclosed above, shows chemical instability, e.g. in the fluids of the digestive system, or if its molecular weight is too high to be absorbed from the gut effectively, transmucosal administration may lead to an improved bioavailability of the compound compared to oral administration. This route of administration is at the same time non-invasive and patient-friendly. Transmucosal administration includes nasal, buccal, sublingual, gingival, and vaginal dosage forms. These dosage forms can be prepared by established techniques; they can be formulated to represent nasal drops or sprays, inserts, films, patches, gels, ointments, or tablets. Preferably, the excipients used for a transmucosal dosage form include one or more substances providing for mucoadhesion, thus prolonging the contact time of the dosage form with the site of absorption and thereby potentially increasing the extent of absorption.

In a further embodiment, the compounds are administered via the pulmonary route, using a metered dose inhaler, a nebuliser, an aerosol spray, or a dry powder inhaler. Appropriate formulations can be prepared by known methods and techniques. Transdermal, rectal, or ocular administration may also be feasible in some cases.

It can be advantageous to use advanced drug delivery or targeting methods in compositions according to the invention more effectively. For instance, if a non-parenteral route of administration is chosen, an appropriate dosage form may contain a bioavailability enhancing agent, which may be any substance or mixture of substances which increases the availability of the compound. This may be achieved, for instance, by the protection of the compound from degradation, such as by an enzyme inhibitor or an antioxidant. More preferably, the enhancing agent increases the bioavailability of the compound by increasing the permeability of the absorption barrier, which is typically a mucosa. Permeation enhancers can act via various mechanisms; some increase the fluidity of mucosal membranes, while others open or widen the gap junctions between mucosal cells. Still others reduce the viscosity of the mucus covering the mucosal cell layer. Among the preferred bioavailability enhancers are amphiphilic substances such as cholic acid derivatives, phospholipids, ethanol, fatty acids, oleic acid, fatty acid derivatives, EDTA, carbomers, polycarbophil, and chitosan.

As mentioned, the compositions comprising gallic acid or derivatives thereof may also be formulated as nutraceutical compositions, i.e. foods and beverages for the purpose of supporting the treatment or prevention of diseases and disorders linked to P-selectin activity according to the present invention. The beverages may include juices such as vegetable (e.g. carrot, and tomato juice) and fruit juice (e.g. orange, apple, grape, and pineapple juice); alcoholic beverages such as beverages based on red, rosé or white wine; teas (e.g. green tea); carbonated beverages; and beverages of supplementary nutrition (e.g. containing vitamins and vitamin complexes). Other nutraceutical compositions in the scope of the invention are sweets (e.g. chocolate and cookies); confectionary; olive oil; food products made from cereal flour like bread, crackers and noodles.

The amount of active ingredient to be incorporated into the compositions in accordance with the invention depends on several factors including the specifically intended use, the target users, and the active compound which is actually selected, i. e. whether it is a polyhydroxy phenol or a polyphenol. In most cases, the desired effects will be achievable with compositions containing 1 microgram of gallic acid or more. In another embodiment, the composition comprises at least 10 mg, and in another embodiment at least 100 or 500 mg. Of course, when derivatives with much higher anti P-selectin activity than gallic acid or (-)-epigallocatechin gallate are used, the dose needs to be adjusted accordingly, such as to less than 100 mg, less than 10 mg, or even less than 1 mg.

The active ingredient may be incorporated in pure form, as is typical for pharmaceutical products. Alternatively, if gallic acid or a natural derivative of gallic acid is used as active ingredient, the active compound may be incorporated in the form of a plant extract. The plant extract may be either liquid or solid-state, and numerous techniques are known and well-established to prepare such an extract. In one of the preferred embodiments, a plant extract is used which has been enriched in gallic acid, or in at least one gallic acid derivative having affinity to P-selectin, which means that the process of preparing the extract has been designed, modified, or optimised to have yield an especially large content of the compound(s) in question. The extract may, for instance, be prepared directly or indirectly from olives, red or white grapes, or green tea. In a preferred embodiment, it is prepared from red wine or red grapes.

The present invention also provides uses for pharmaceutical and nutraceutical compositions containing a polyhydroxy phenol or a polyphenol with affinity to P-selectin. The uses are consistent with the methods of binding or inhibiting the activity of P-selectin as described above, and include the diagnosis, prevention, or treatment of a disease or condition selected from coronary artery disease, thrombosis, cancer, chronic inflammatory disorders, rheumatoid arthritis, inflammatory bowel disease, multiple sclerosis, atherosclerosis, restenosis, ischemia, reperfusion injury including renal failure, tumour metastasis, bacterial sepsis, disseminated intravascular coagulation, adult respiratory stress syndrome, stroke, angiogenesis, transplant rejection, deep vein thrombosis, myocardial infarction or circulatory shock.

According to the diagnostic use, the compositions may be employed for in vitro tests to quantify P-selectin concentrations in body fluids and tissues (arterial lesions) as markers for the diseases and conditions associated with P-selectin. They may be also used for in vivo diagnostic imaging procedures to monitor P-selectin mediated atherosclerosis, restenosis, and other conditions selected from those in which P-selectin is mobilized. As an option for this use, a gallic acid molecule or derivative according to the invention may be conjugated with a chelator, which is subsequently complexed with an isotropic label that is detectable by the chosen monitoring system or associated with a contrast agent (DTPA chelated gadolinium or USPIO).

The following examples are intended to illustrate certain aspects and embodiments of the invention; however, they are not to be understood as to limit the scope of the claims as set forth below.

### EXAMPLES

### Example 1: Competition assay of gallic acid with TM11-PO

Gallic acid (Acros, Geel, Belgium) was assayed for its ability to inhibit TM11-PO binding to human P-selectin. TM11-PO, a tetrameric TM11/strepPO complex, which has previously been shown to bind with high affinity and specificity to human P-selectin (Molenaar et al., Blood 2002, 100, 3570-3577), was freshly prepared by incubating streptavidin-peroxidase (strep-PO (Amersham Life Science, Little Chalfont, United Kingdom), 8.4 µl, 2.0 µM) and TM11-biotin (biotin-CDVEWVDVSSLEWDLPC (synthesised by Dr. Van der Zee, Department of Immunology, University of Utrecht, Utrecht, the Netherlands), 1.5 µl 190 mM) for 2 hours at room temperature in assay buffer (20 mM HEPES, 150 mM NaCl, 1 mM CaCl₂, pH 7.4). For competition studies, a 96 wells microtiter plate (high binding, flat bottom, Costar, Corning, U.S.A.) was coated overnight at 4°C with 10 µg/ ml goat anti-human IgG (Fc specific) (Sigma-Aldrich, Zwijndrecht, the Netherlands) in coating buffer (50 mM NaHCO₃, pH 9.6). Subsequently, wells were washed with assay buffer and incubated for 1 hour at 37°C with blocking buffer (3% BSA in assay buffer). After washing with assay buffer, the wells were incubated for 2 hours at 37°C with the human P-selectin IgG-Fc chimera (R&D Systems Europe Ltd., Abingdon, United Kingdom) (0.3 µg/ml). Subsequently, wells were washed with assay buffer and incubated for 1 hour at 4°C with the TM11-PO complex. The wells were washed six times with washing buffer (0.1% Tween 20 in assay buffer). 3,3',5,5'-Tetramethylbenzamidine (TMB)/hydrogen peroxide (H₂O₂) (Pierce, Rochford, U.S.A.) was added and wells were incubated at room temperature for 15 minutes. The reaction was halted by addition of 2 M H₂SO₄ and the absorbance was measured at 450 nm.

In result, gallic acid was found to be a potent inhibitor of TM11-PO binding to human P-selectin with an IC₅₀ value of 7.2 µM.

### Example 2: Competition assay of gallic acid (derivatives) and polyphenols with PAA-Le^{a}-SO₃H

To be able to test gallic acid binding also to other members of the selectin family (i.e. E- and L-selectin), biotin-PAA-Le^{a}-SO₃H (Synthesone, Munich, Germany) instead of TM11-PO was used as ligand in the competition assay. This polyacrylamide (PAA) based polymer with attached sulfo-Lewis A groups, is an established selectin ligand.

Experimentally, the same procedure was used as in example 1 however with the following differences. After washing with assay buffer, the wells were incubated for 2 hours at 37°C with the human P-selectin IgG-Fc chimera, mouse P-selectin IgG-Fc chimera, human L-selectin IgG-Fc chimera and human E-selectin IgG-Fc chimera respectively (all from R&D Systems Europe Ltd., Abingdon, United Kingdom) (0.3 µg/ml). Subsequently the wells of the microtiter plate were incubated with biotin-PAA-Le^{a}-SO₃H (0.33 µg/ml) for 2 hours at 37°C, instead of with the TM11-PO-complex.

Gallic acid in this test was found to inhibit human P-selectin (IC₅₀ of approx. 85 µM). It was further found that gallic acid could not inhibit E-selectin binding, showing less than 20% inhibition at concentrations up to 1 mM, whereas it appeared to be a moderate inhibitor of L-selectin, with an IC₅₀ of 241 µM. Gallic acid binding to P-selectin appeared to be species-independent.

In addition to gallic acid itself, some derivatives of gallic acid (n-dodecyl gallate (Lancaster, Morecambe, United Kingdom) and (-)-epigallocatechin gallate (EGCG) (Sigma-Aldrich, Zwijndrecht, the Netherlands)), a polyphenol having anti-oxidant properties (caffeic acid) (Sigma-Aldrich, Zwijndrecht, the Netherlands) and 4-hydroxy benzoic acid (Acros, Geel, Belgium) were tested for their ability to inhibit human P-selectin. Of these compounds, EGCG was able to inhibit biotin-PAA-Le^{a}-SO₃ binding to human P-selectin having an IC₅₀ of 114 µM. All other compounds showed IC₅₀ -values of > 300 µM.

### Example 3: Inhibition of dynamic interactions between HL60 cells and human P-selectin expressing Chinese hamster ovary cells

Chinese hamster ovary (CHO) cells stably transfected with human P-selectin (CHO-P) were kindly donated by Dr. Modderman (University of Amsterdam, Amsterdam, the Netherlands). Cells were grown in DMEM (BioWhittaker Europe, Verviers, Belgium) containing 10% foetal calf serum (FCS) (Bio Witthaker), 5 mM L-glutamine, 20,000 units penicillin/streptomycin (Bio Whittaker) and 5 mM non-essential amino acids (Gibco, Paisley, United Kingdom). Flasks with cells were incubated at 37°C in 5% CO₂ for 3 or 4 days until cells had grown nearly confluent.

HL60 cells were from ATCC and grown in RPMI 1640 medium (BioWhittaker) with 10% FCS, 5mM L-glutamine and 20,000 units penicillin/streptomycin.

Dynamic interactions between macrophage-derived HL60 cells, displaying a high expression of PSGL-1, the natural P-selectin ligand, and human P-selectin expressing Chinese hamster ovary cell (CHO-P cells) monolayers grown onto glass coverslips coated with 30 µg/ml collagen S (type I) (Roche Diagnosis, Brussels, Belgium) were analysed in a parallel-plate perfusion chamber with a method adapted from G. Theilmeier (Blood 1999, 94: 2725-2734). The coverslip constituted the bottom of the chamber and the actual chamber was formed by a 254-µm height silicon rubber gasket designed with a conically shaped flow path, thus resulting in a 3-fold increase of wall shear rate from the inlet of the chamber to the outlet. Calcein-AM labeled HL60 cells, suspended in RPMI (0.5 x 106/ml) were perfused at 37°C and at a flow rate of 1 ml/min with an inverted syringe pump (Harvard Instruments, South Natick, MA, U.S.A.). The flow chamber was mounted on the table platform of an inverted epifluorescence microscope (Diaphot; Nikon, Melville, NY, U.S.A.) coupled to a Cohu CCD video camera (COHU Inc, San Diego, CA, U.S.A.) and HL60 cell translocation over CHO-P monolayers were measured at wall shear rates of 300 and 600 s-1. Gallic acid was added to HL60 cell suspensions 2 minutes before the onset of perfusion. To prevent oxidation, the gallic acid solution was prepared freshly. Real time movies of 12 seconds (10 images per second), recorded at predefined positions in the flow path corresponding to chosen wall shear rates were stored and digitised with a Scion LG3 frame grabber (Scion Corp, Frederick, ML, U.S.A.). The average velocity of HL60 cells rolling over the CHO-P cells was calculated from the rolling distance of the HL60 cells in a 1 second timeframe, using the NIH Image program version 6.1. The number of adhered HL60 cells was counted from pictures taken during the same experiment.

As a result, gallic acid significantly decreased HL60 cell adhesion to the CHO-P monolayer already at 50 µM as compared to the untreated control, irrespective of the wall shear rates (300 s⁻¹ and at 600 s⁻¹, -34% and -43% respectively). At 250 µM, the effect of gallic acid was even more pronounced (-41 % and -54% respectively).

The rolling velocity of the HL60 cells rolling across the CHO-P monolayer was measured in the same experimental setup. The rolling velocity of HL60 cells was doubled in the presence of 50 and 250 µM of gallic acid.

## Claims

1. Use of a polyhydroxy phenol or a polyphenol for the manufacture of a medicament for the prevention, treatment or diagnosis of a disease or condition, wherein P-selectin is involved, in a subject.

2. Use according to claim 1, **characterised in that** the prevention, treatment or diagnosis of the disease or condition is based on inhibition of P-selectin.

3. Use according to claim 1 or 2, **characterised in that** the polyhydroxy phenol or polyphenol is present as a multimer.

4. Use according to any one of claims 1 to 3, **characterised in that** the polyhydroxy phenol is selected from the group consisting of gallic acid, n-dodecyl gallate, caffeic acid and 3,4,5-trihydroxy cinnamic acid.

5. Use according to any one of claims 1 to 3, **characterised in that** the polyphenol is selected from the group consisting of (-)-epigallocatechin gallate, (epi)catechin, m-galloyl gallic acid and ellagic acid.

6. Use according to any one of claims 1 to 5, **characterised in that** the disease or condition in which P-selectin is involved is selected from the group consisting of coronary artery disease, thrombosis, cancer, chronic inflammatory disorders, rheumatoid arthritis, inflammatory bowel disease, multiple sclerosis, atherosclerosis, restenosis, ischemia, reperfusion injury including renal failure, tumour metastasis, bacterial sepsis, disseminated intravascular coagulation, adult respiratory stress syndrome, stroke, angiogenesis, transplant rejection, deep vein thrombosis, myocardial infarction or circulatory shock.

7. Use according to any one of claims 1 to 6, **characterised that** a human being is suffering from the disease or condition in which P-selectin is involved.

8. Use according to any one of claims 1 to 7, **characterised in that** the polyhydroxy phenol or the polyphenol is administered orally or parenterally.

9. Use according to any one of claims 1 to 8, **characterised in that** gallic acid is administered in a selected amount which is sufficient to obtain concentrations of 1 µM, and preferably of at least 5 µM at the site of action.

10. Use according to any of claims 1 to 9, **characterised in that** the polyhydroxy phenol or the polyphenol is administered in a selected amount which is sufficient to obtain a P-selectin inhibition which is at least equivalent to the P-selectin inhibition achieved by gallic acid concentrations of 1 µM, and preferably of 5 µM at the site of action.

11. Use according to any of claims 1 to 10, **characterised in that** from 1 microgram to 10g of the polyhydroxy phenol or polyphenol is administered per day.

12. Use of a polyhydroxy phenol or a polyphenol having affinity to P-selectin as a targeting agent to P-selectin-expressing cells or tissues.

13. Use according to claim 12, **characterised in that** the polyhydroxy phenol or polyphenol is incorporated into a drug-delivery system, comprising an active compound in a vehicle.

14. Use according to claim 12 or 13, **characterised in that** the polyhydroxy phenol or the polyphenol is coupled to the active compound.

15. Use according to any one of claims 12 to 14, **characterised in that** the vehicle is a polymer, a hydrogel, a liposome, a nanoparticle, a microparticle, a complex, or a virosome.

16. A pharmaceutical or neutraceutical composition, comprising a polyhydroxy phenol or a polyphenol.

17. Composition according to claim 16, comprising (a) an active ingredient selected from gallic acid, a gallic acid derivative having affinity to P-selectin, a plant extract containing gallic acid and/or a gallic acid derivative having affinity to P-selectin, or a mixture thereof, and (b) at least one pharmaceutical excipient.

18. The composition according to claim 16 or 17, wherein the active ingredient is a dry or liquid plant extract which is enriched in gallic acid and/or a gallic acid derivative having affinity to P-selectin.

19. The composition according to claim 18, wherein the plant extract is prepared from red wine or grapes.

20. The composition according to any of claims 16 to 19, which is formulated and processed for oral administration, preferably in form of a liquid, a tablet, a capsule, granules, an enteric solid dosage form, a solid dosage form providing sustained or controlled release, an effervescent tablet, or an orally disintegrating dosage form.

21. The composition according to any of claims 16 to 20, further comprising a bioavailability enhancing agent and/or a stabilising antioxidant.
